# EUROPEAN PATENT SPECIFICATION

(11) **EP 4 132 571 B1**
(45) Date of publication and mention of the grant of the patent: **29.10.2025**
(21) Application number: 21784796.1
(22) Date of filing: 07.04.2021
(51) Int. Cl.: A61K 31/05, A61K 31/20, A61K 39/00, A61K 39/02, A61K 39/12, A61K 31/201, A61K 31/202, A61K 31/353, A61K 38/07, A61K 38/08, A61K 38/12, A61P 11/06, A61P 37/06, A61K 31/365, A61K 31/575, A61K 35/60

(54) **SALMONID OIL COMPOSITIONS FOR USE IN THE TREATMENT OF EOSINOPHILIC RELATED INFLAMMATORY AND RESPIRATORY CONDITIONS**
SALMONIDÖLZUSAMMENSETZUNGEN ZUR VERWENDUNG BEI DER BEHANDLUNG VON MIT EOSINOPHILEN IN ZUSAMMENHANG STEHENDEN ENTZÜNDLICHEN UND RESPIRATORISCHEN ZUSTÄNDEN
COMPOSITIONS D'HUILE DE SALMONIDÉ POUR UNE UTILISATION DANS LE TRAITEMENT D'ÉTATS INFLAMMATOIRES ET RESPIRATOIRES EN RAPPORT AVEC LES ÉOSINOPHILES

(30) Priority: 07.04.2020 US 202063006327 P; 17.11.2020 US 202063114960 P
(43) Date of publication of application: 15.02.2023
(73) Proprietor: Hofseth Biocare ASA, 6003 Ålesund (NO)
(72) Inventor: FRAMROZE, Bomi, 6003 Ålesund (NO)
(74) Representative: Zacco Norway AS
(86) International application number: PCT/US2021/026193
(87) International publication number: WO 2021/207367

(56) References cited:
- WO-A1-2018/222173
- WO-A1-2019/245380
- WO-A1-2019/245380
- US-A1- 2009 269 321
- MICKLEBOROUGH TIMOTHY D. ET AL: "Protective Effect of Fish Oil Supplementation on Exercise-Induced Bronchoconstriction in Asthma", CHEST, vol. 129, no. 1, 1 January 2006 (2006-01-01), US, pages 39 - 49, XP093108569, ISSN: 0012-3692, DOI: 10.1378/chest.129.1.39
- FRAMROZE BOMI, HEGGDAL HENRIETTE: "An in vitro study to explore the modulation of eosinophil effector function in human allergic peripheral blood eosinophils using enzymatically extracted salmonid oil", FUNCTIONAL FOODS IN HEALTH AND DISEASE, vol. 10, no. 8, 31 August 2020 (2020-08-31), pages 357 - 367, XP055865216, ISSN: 2378-7007, DOI: 10.31989/ffhd.v10i8.730

## Description

This application claims priority to and the benefit of U.S. Provisional Application Nos. 63/006,327, filed on April 7, 2020, and 63/114,960, filed on November 17, 2020.

### FIELD

The present disclosure relates generally to respiratory treatments, and more specifically to the use of salmonid oil products to treat respiratory conditions, diseases or disorders, such as asthma, in a subgroup of patients that exhibit resistance to steroid therapies.

Any references in the description to methods of treatment refer to the compounds, pharmaceutical compositions and medicaments of the invention for use in a method of treatment of the human body by therapy.

### BACKGROUND

Inflammation is an immunological conundrum. On the one hand, the physiological changes that accompany inflammation allow us to mount an acute response to external threats that would otherwise have wiped out the human species. On the other, chronic inflammation, where age or external stressors keep our immune system in overdrive, can contribute to many debilitating diseases ranging from Alzheimer's to diabetes and bronchial asthma.

An eosinophil is a type of white blood cell stored in tissues throughout the body and continually replenished from the bone marrow. Eosinophils typically have a two-day lifespan in blood, but inflammatory conditions such as infections and allergic diseases will extend the lifespan up to two weeks by eosinophil-activating cytokines. *See* Park YM & Bochner BS, Allergy Asthma Immunol Res. 2010, 2:87-101. An eosinophil count is a blood test that measures the quantity of eosinophils in the human body. Elevated levels, usually measured during routine complete blood count testing, indicate an infection or allergy.

Activated eosinophils, which are promoted by eosinophil-activating cytokines under inflammatory conditions, are a major source of reactive oxygen species, cytotoxic proteins and proinflammatory cytokines. They signal the activation of resident tissue cells such as epithelial, endothelial and fibroblast cells, leading to the progression of inflammation and mucus secretion. Eosinophils are therefore potent activators and modulators of diseases such as bronchial asthma, atopic dermatitis' and colitis ulcerosa. *See* Hogan SP. Int Arch Allergy Immunol. 2007, 143(Suppl 1):3-14; Simon D et al., Allergy. 2004, 59:561-570; Wedemeyer J & Vosskuhle K., Best Pract Res Clin Gastroenterol. 2008, 22:537-549; and Mickkleborough T. D. et al., Chest. 2006, 129(1):39-40.
Further, in asthmatics, levels of eosinophil granule proteins such as eosinophil cationic protein (ECP) and eosinophil peroxidase (EPO) largely correlate with asthma severity. *See* Parra A, et al., J Investig Allergol Clin Immunol. 1999; 9:27-34. Eosinophilic inflammation of the upper airways may also occur independent of allergy, as observed in chronic rhinosinusitis (CRS) subjects. *See* Hutcheson PS, et al., J Rhinol Allergy. 2010, 24:405-408. Such individuals represent a unique subgroup who are largely resistant to medical and surgical interventions and who could show immediate benefit by therapy that targets eosinophilic expansion and effector functions.

### BRIEF SUMMARY

In some aspects, provided herein respiratory treatments, such as asthma treatments, using salmonid oil. In some embodiments, the asthma is bronchial asthma. In certain embodiments, the treatments provided target a subgroup of patients that are largely resistant to medical and surgical interventions, including steroid therapies.

In certain aspects, provided is a method for treating an inflammatory condition, disorder or disease in a human in need thereof, comprising: administering to the human an effective dose of a composition comprising salmonid oil or at least one biological active isolated from salmonid oil to treat the inflammatory condition, disorder or disease.

In certain aspects, provided is a method for treating a respiratory condition, disorder or disease in a human in need thereof, comprising: administering to the human an effective dose of a composition comprising salmonid oil or at least one biological active isolated from salmonid oil to treat the inflammatory condition, disorder or disease.

In some variations of the methods provided herein, the condition, disorder or disease treated is an eosinophilic condition, disorder or disease. In some embodiments, an inflammatory condition, disorder or disease is an eosinophilic inflammatory condition, disorder or disease. In some embodiments, a respiratory condition, disorder or disease is an eosinophilic respiratory condition, disorder or disease.

In certain aspects, provided is a method for reducing eosinophil effector function in a human in need thereof, comprising administering to the human a composition comprising salmonid oil or at least one biological active isolated from salmonid oil to reduce eosinophil effector function.

In some embodiments of the foregoing aspects, the salmonid oil is enzymatically extracted salmonid oil. In certain variations, the salmonid oil is obtained from mild enzymatic hydrolysis of off-cuts of salmonid fish.

In some embodiments of the methods provided herein, the composition is administered orally. In some variations, the composition is inhaled. In some variations, the composition is administered nasally. In some variations, the composition is injected. In some variations, the composition is administered topically.

In certain aspects, provided are also salmonid oil compositions comprising at least one biological active described herein. In certain aspects, provided are compositions comprising at least one biological active isolated from salmonid oil. In another aspect, provided is the use of such compositions for treating the various conditions, disorders or diseases described herein.

In other aspects, provided is an article of manufacture, comprising: a container comprising a composition comprising salmonid oil or at least one biological active isolated from salmonid oil; and a label containing instructions for use of such composition.

In yet other aspects, provided is a kit, comprising: a dosage form of a salmonid oil composition or a composition comprising at least one biological active isolated from a salmonid oil composition: and a package insert containing instructions for use of such composition.

In some variations of the foregoing aspects, the dosage form is a syrup, chewable, capsule or soft gel.

### DESCRIPTION OF THE FIGURES

The present application can be understood by reference to the following description taken in conjunction with the accompanying figures.
FIGS. **1** and 3 depict shape change (100% as baseline) of PMNL as a function of CCL11 concentration, with different pretreatment conditions.
FIGS. **2** and **4** depict CB11b change (100% as baseline) on PMNL surface as a function of CCL11 concentration, in different pretreatment conditions.

### DETAILED DESCRIPTION

The following description sets forth exemplary methods, parameters and the like. It should be recognized, however, that such description is not intended as a limitation on the scope of the present disclosure but is instead provided as a description of exemplary embodiments.

In some aspects, provided herein are methods for treating inflammatory conditions, disorders or diseases in humans in need thereof by orally administering salmonid oil compositions.

### Salmonid Oil Compositions, and Methods of Producing Thereof

In some embodiments, the compositions administered in the methods provided herein are salmonid oil compositions. Salmonids is a family of fish in the order Salmoniformes, and includes, for example, salmon, trout, chars, freshwater whitefishes and graylings.

In some aspects, the salmonid oil compositions comprise enzymatically extracted salmonid oil. In some variations, the enzymatically extracted salmonid oil is obtained from mild enzymatic hydrolysis of off-cuts of salmonid fish. In some variations, the salmonid oil compositions are natural, unrefined and gently liberated from salmonid. In some variations, the process to produce the salmonid oil compositions does not use, or does not require using, chemicals, solvents, high pressure or heat. Methods for obtaining enzymatically extracted salmonid oil are disclosed in WO 2019/245380 A1.

The salmonid oil compositions used in the methods described herein are prepared by mild enzymatic hydrolysis, which does not require harsh chemicals, fractional distillation, reesterification, winterization, high pressure, and/or heat. For example, in some variations, the salmonid oil compositions used in the methods described herein are prepared by enzymatic hydrolysis that does not require the use of strong acids or bases (e.g., including 3 N or higher acid or base), fractional distillation, reesterification, winterization, pressures of at least 30 psi, or heat at temperatures of at least 130°C. The use of the mild enzymatic hydrolysis conditions described herein are generally milder than techniques often used in the art to produce salmonid oil. *e.g*., fractional distillation or "cold-pressed" processing, which include the use of strong acid or base, fractional distillation, reesterification, winterization, high pressure, and/or heat. When mild enzymatic hydrolysis as described herein is used, the resulting composition has biologically active components that are surprisingly beneficial to treating conditions related to eosinophil or eosinophil dysfunction.

In some embodiments, the enzymatic hydrolysis process to produce the salmonid oil compositions uses less than 3 N, less than 2.5 N, less than 2 N, less than 1.5 N, less than 1 N. less than 0.5 N, less than 0.1 N, less than 0.01 N, or less than 0.001 N acid. In some embodiments, the enzymatic hydrolysis process does not use or does not require using any additional acid. In some variations of the foregoing, the acid is a strong acid. In some variations of the foregoing, the acid is HCl or HNO₃, or any combination thereof.

In some embodiments, the process to produce the salmonid oil compositions uses less than 3 N, less than 2.5 N, less than 2 N, less than 1.5 N. less than 1 N, less than 0.5 N, less than 0.1 N, less than 0.01 N, or less than 0.001 N base. In some embodiments, the process does not use or does not require using any additional base. In some variations of the foregoing, the base is a strong base (such as NaOH and/or KCl).

In some embodiments, the salmonid oil obtained from mild enzymatic hydrolysis is not mixed or does not require mixing with an acid of 3 N or higher, 2.5 N or higher, 2 N or higher, 1.5 N or higher, 1 N or higher, 0.5 N or higher, 0.1 N or higher, 0.01 N or higher, or 0.001 N or higher concentration. In some variations of the foregoing, the acid is a strong acid (such as HCl and/or HNO₃).

In some embodiments, the salmonid oil obtained from mild enzymatic hydrolysis is not mixed or does not require mixing with a base of 3 N or higher, 2.5 N or higher, 2 N or higher, 1.5 N or higher, 1 N or higher, 0.5 N or higher, 0.1 N or higher, 0.01 N or higher, or 0.001 N or higher concentration. In some variations of the foregoing, the base is a strong base (such as NaOH and/or KOH).

In some embodiments, less than 3 mmol, less than 2.5 mmol, less than 2 mmol, less than 1.5 mmol, less than 1 mmol, less than 0.5 mmol, less than 0.1 mmol, less than 0.01 mmol, or less than 0.01 mmol of acid or H⁺ is added per gram of enzymatically extracted salmon oil. In some embodiments, no acid or H⁺ is added or required to be added to the enzymatically extracted salmon oil.

In some embodiments, less than 3 mmol, less than 2.5 mmol, less than 2 mmol, less than 1.5 mmol, less than 1 mmol, less than 0.5 mmol, less than 0.1 mmol, less than 0.01 mmol, or less than 0.01 mmol base or OH⁻ is added per gram of enzymatically extracted salmon oil, less than 6 mmol. In some embodiments, no base or OH⁻ is added or required to be added to the enzymatically extracted salmon oil.

In some embodiments, no acid is added or required to be added during or after enzymatic hydrolysis to produce the salmonid oil used in the methods herein. In some embodiments, less than 3 N, less than 2.5 N, less than 2 N. less than 1.5 N. less than 1 N, less than 0.5 N, less than 0.1 N, less than 0.01 N, or less than 0.001 N acid is added during or after enzymatic hydrolysis. In some variations of the foregoing, the acid is a strong acid (such as HCl and/or HNO₃).

In some embodiments, no base is added or required to be added during or after enzymatic hydrolysis to produce the salmonid oil used in the methods herein. In some embodiments, less than 3 N, less than 2.5 N, less than 2 N, less than 1.5 N, less than 1 N, less than 0.5 N, less than 0.1 N, less than 0.01 N, or less than 0.001 N base is added during or after enzymatic hydrolysis. In some embodiments, the base is a strong base (such as NaOH and/or KOH).

In some embodiments, the process to produce the salmonid oil compositions is performed at a temperature below below 130°C, below 120°C, below 110°C, below 100°C, below 90°C, below 80°C. below 70°C. below 60°C. below 55°C. below 30°C, below 45°C. below 40°C, below 35°C, below 30°C, or below 25°C. In some embodiments, the temperature during or after enzymatic hydrolysis is maintained below 130°C, below 120°C, below 110°C, below 100°C, below 90°C, below 80°C, below 70°C, below 60°C, below 55°C. below 50°C, below 45°C, below 40°C, below 35°C, below 30°C, or below 25°C.

In some embodiments, the salmonid oil obtained from mild enzymatic hydrolysis is not exposed or required to be exposed to a temperature of 150°C or higher, 140°C or higher, 130°C or higher, 120°C or higher, 110°C or higher. 100°C or higher, (X)°C or higher, 80°C or higher, 70°C or higher. 60°C or higher. 55°C or higher. 50°C or higher. 45°C or higher, 40°C or higher, 35°C or higher, 30°C or higher, or 25°C or higher.

In some embodiments, the process to produce the salmonid oil compositions is performed at a pressure below 2 atm, below 1.5 atm, below 1.4 atm, below 1.3 atm, below 1.2 atm, below 1.1 atm, or at or around 1.0 atm. In some embodiments, the process to produce the salmonid oil compositions is performed at a pressure below 30 psi, below 25 psi, below 20 psi, below 15 psi, or at or around 14.7 psi.

In some embodiments, pressure during or after enzymatic hydrolysis is maintained below 2 atm, below 1.5 atm, below 1.4 atm, below 1.3 atm, below 1.2 atm, below 1.1 atm, or at or around 1.0 atm. In some embodiments, pressure during or after enzymatic hydrolysis is maintained below 30 psi, below 25 psi, below 20 psi, below 15 psi, or at or around 14.7 psi.

In some embodiments, the enzymatically extracted salmon oil is not exposed to or required to be exposed to more than 2 atm, more than 1.5 atm, more than 1.4 atm, more than 1.3 atm, more than 1.2 atm, more than 1.1 atm, or more than 1.0 atm. In some embodiments, the enzymatically extracted salmon oil is not exposed to or required to be exposed to more than 30 psi, more than 25 psi, more than 20 psi, more than 15 psi, or more than 14.7 psi.

In some embodiments, salmonid oil provided herein is produced without using one or more of the following processes or conditions: (i) mixing with 3 N or higher concentration of acid; (ii) mixing with 3 N or higher concentration of base; (iii) fractional distillation: (iv) reesterification; (v) winterization; (vi) 130°C or higher temperature, or (vi) 2 atm or higher pressure. In some variations, salmonid oil provided herein is produced without using any of the above-listed processes or conditions.

In certain variations, the process to obtain the salmonid oil compositions comprises the use of protease enzymes that contain less than certain percentage (e.g., less than 1% or less than 0.5%) relative lipase activity, offcut raw materials that are less than certain hours old from slaughter (e.g., less than 24 hours old from slaughter), and/or turbine mixing during enzymatic hydrolysis. This allows for the quantitative extraction of all the lipid components found in the whole fish, with minimum to no loss of minor, non-fatty acid biologically active constituents.

Further, using enzymes with low lipase activity minimizes the amount of free fatty acid molecules in the mixture, where these free fatty acid molecules could degrade biologically active constituents and/or shorten shelf life of the salmonid oil with their prooxidative properties. Thus, using enzymes with low lipase activity, offcut raw materials that are relatively fresh, and turbine mixing to shorten the time period for enzymatic hydrolysis, all contribute to producing salmonid oil with maximal effect on eosinophilic functions.

In some embodiments, the process to produce the salmonid oil compositions comprises the use of enzymes that contain less than 3%, less than 2%, less than 1%, less than 0.9%, less than 0.8%, less than 0.7%, less than 0.6%, less than 0.5%, less than 0.4%, less than 0.3%, less than 0.2%, less than 0.15%, less than 0.1%, less than 0.05%, or less than 0.01% lipase activity. In some embodiments, mild enzymatic hydrolysis of off-cuts of salmonid fish comprises the use of enzymes with less than 3%, less than 2%, less than 1%, less than 0.9%, less than 0.8%, less than 0.7%, less than 0.6%, less than 0.5%, less than 0.4%, less than 0.3%, less than 0.2%, less than 0.15%, less than 0.1%, less than 0.05%, or less than 0.01% lipase activity.

In some embodiments, the enzymatically extracted salmonid oil comprises less than 3%, less than 2.5%, less than 2%, less than 1.5%, less than 1%, less than 0.9%, less than 0.8%, less than 0.7%, less than 0.6%, less than 0.5%, less than 0.4%, less than 0.3%, less than 0.2%, less than 0.1%, less than 0.05%, or less than 0.01% free fatty acid. In some embodiments, the enzymatically extracted salmonid oil comprises less than 3%, less than 2.5%, less than 2%, less than 1.5%, less than 1%, less than 0.9%, less than 0.8%, less than 0.7%, less than 0.6%, less than 0.5%, less than 0.4%, less than 0.3%, less than 0.2%, less than 0. 1%, less than 0.05%, or less than 0.01% free fatty acid without going through winterization or reesterification. In some embodiments, the enzymatically extracted salmonid oil comprises less than 0.5% free fatty acid without going through winterization or reesterification.

In some embodiments, the enzymatically extracted salmonid oil described herein is prepared from salmon that is less than 1 hour, less than 2 hours, less than 3 hours, less than 4 hours, less than 5 hours, less than 6 hours, less than 7 hours, less than 8 hours, less than 9 hours, less than 10 hours, less than 11 hours, less than 12 hours, less than 24 hours, less than 36 hours, less than 48 hours, between 2-6 hours, between 4-8 hours, between 6-10 hours, or between 2-10 hours from slaughter of the salmon.

In one embodiment, the salmonid oil composition is OmeGo^{®} Salmon Oil, Cardio^{®} Salmon Oil, or Brilliant^{®} Salmon Oil, each of which are variations of enzymatically extracted salmon oil commercially available from Hofseth BioCare ASA.

It was unexpectedly observed that common fatty acids found in fish oils in triglyceride or phospholid forms were not responsible for reducing eosinophil effector function. Rather, it is the presence of certain minor, biologically active constituents present in the salmonid oil compositions described herein that have such effect.

In certain embodiments, the biologically active constituent(s) in the salmonid oil compositions include minor fatty acid triglyceride components. In some variations, the biologically active constituent(s) include saturated acids. In certain variations, the biologically active constituent(s) include caproic acid, caprylic acid, capric acid, lauric acid, behenic acid, or lignoceric acid, or any combination thereof.

In some variations, the biologically active constituent(s) in the salmonid oil compositions include monounsaturated acids. In certain variations, the biologically active constituent(s) include myrstoleic acid, heptadecenoic acid, elaidic acid, gadoleic acid, erucic acid, brassidic acid, and/or nervonic acid.

The biologically active constituent(s) include gamma linolenic acid, columbinic acid, stearidonic acid, mead acid, and/or dihomo gamma linolenic acid.

In other variations, the biologically active constituent(s) in the salmonid oil compositions include small organic molecule(s). In some variations, the biologically active constituent(s) include terpenes *(e.g..* ligustilide), sesquiterpenes *(e.g.,* germacrene), phenols (*e.g.,* thymol, eugenol, carvacrol), alcohols (*e.g..* linalool, citronellol, terpineol), sesquiterpene alcohols (*e.g*., bisbalol, santalol), ketones (*e.g*., thujone, pinacamphone, italidone), esters (*e.g*., linalyl acetate, geranyl acetate, citronellyl formate), lactones and coumarins (*e.g*., helenalin, elecampane, furocoumarin), ethers (*e.g*., chavicol). steroid derivatives (*e.g.,* sitosterol, stigmasterol), and/or phthalide derivatives (*e.g..* 3-butyliden-4.5-dihydrophthalide).

In some variations, the biologically active constituent(s) in the salmonid oil compositions include lipopeptide(s). In some variations, the biologically active constituent(s) include linear and/or cyclic lipopeptides. In certain variations, the biologically active constituent(s) include iturin A, hoiamides, heronamides, laxaphycin, apramides, dragonamides, gageotetrins, lyngkyabellins, cyclodycidins, parguerine, pumilacidin, sulforeido lipopeptides, fengycins, mebamamides, microcolins, penicimutamides, sulfoglycolipids, halovir, kahalalide, and/or tuftsin.

In some variations, the biologically active constituent(s) in the salmonid oil compositions include linear lipopeptides. In certain variations, the biologically active constituent(s) in the salmonid oil compositions include microcolin A. In one variation, the salmonid oil compositions comprise microcolin A at a concentration of at least 5 µg/mL, at least 10 µg/mL, at least 20 µg/mL, or at least 25 µg/mL, or between 5 µg/mL to 100 µg/mL, between 10 µg/mL and 100 µg/mL, or between 20 µg/mL and 75 µg/mL,

In other variations, the biologically active constituent(s) in the salmonid oil compositions include protectin(s).

In yet other variations, the biologically active constituent(s) in the salmonid oil compositions include lipoxin(s).

In some variations of the foregoing biologically active constituent(s) described, the compound(s) may be present in salt form. In certain variations, the compound(s) may be present in any combinations thereof.

### Conditions, Diseases or Disorders

The salmonid oil compositions provided herein are for use in the treatment of an eosinophilic inflammatory condition, disorder or disease or an eosinophilic respiratory condition, disorder or disease, as disclosed in the appended claims.

In some variations, "treatment" or "treating" is an approach for obtaining beneficial or desired results including clinical results. Beneficial or desired clinical results may include one or more of the following:
(i) decreasing one more symptoms resulting from the condition, disease or disorder:
   (ii) diminishing the extent of the disease and/or stabilizing the condition, disease or disorder (e.g., delaying the worsening of the condition, disease or disorder):
   (iii) delaying the spread of the condition, disease or disorder,
   (iv) delaying or slowing the recurrence of the condition, disease or disorder and/or the progression of the condition, disease or disorder;
   (v) ameliorating the disease state and/or providing a remission (whether partial or total) of the condition, disease or disorder and/or decreasing the dose of one or more other medications required to treat the condition, disease or disorder,
   (vi) increasing the quality of life; and/or
   (vii) prolonging survival.

The salmonid oil compositions provided herein may be used to treat asthma, pneumonia, bronchiectasis, emphysema, tuberculosis, lung collapse, lung fibrosis, fibrosing alveolitis, chronic obstructive pulmonary disease (COPD), allergic rhinitis, chronic rhinosinusitis (CRS), and/or acute respiratory disease syndrome.

The condition, disease or disorder is a chronic inflammatory disorder. In certain embodiments, the chronic inflammatory disorder is a chronic inflammatory disorder of the airways. In certain variations, the condition, disease or disorder is an inflammatory lung disease. In some variations, the condition, disease or disorder involves narrowing and/or swelling of airways, thereby making breathing difficult and triggering coughing, wheezing and/or shortness of breath. In certain variations, the condition, disease or disorder is asthma. In certain variations, the asthma is bronchial asthma. In one variation, the condition, disease or disorder involves steroid treatment resistant asthma and airway constrictions.

The condition, disease or disorder is an allergy or an allergic inflammation.

The condition, disease or disorder is a viral respiratory disease. In some variations, condition, disease or disorder is severe acute respiratory syndrome. In certain variations, the severe acute respiratory syndrome is caused by a coronavirus.

In some variations, the human in need thereof is a lung-compromised individual. In certain variations, the lung-compromised individual has fluid build-up in the alveoli in the lungs. This fluid can leak from the smallest blood vessels in the lungs into the alveoli due to the destruction of the protective membrane in the alveoli. The membrane which normally keeps this fluid in the vessels may be destroyed because of a disruption in immune response due to severe disease or injury. The fluid enters the alveoli and keeps the lungs from filling with enough air, which means less oxygen reaches the bloodstream. This deprives organs of the oxygen that is needed to function, which can cause multiple organ failure resulting in death.

Disclosed therein is a method for treating hospitalized lung-compromised humans in need thereof, comprising administering the salmonid oil compositions provided herein, or compositions comprising biological active(s) isolated from the salmonid oil compositions provided herein, as disclosed in the appended claims, to the human to reduce or delay the need to provide the human with assisted respiration.

In some variations, "delaying" development of a condition, disease or disorder means to defer, hinder, slow, retard, stabilize and/or postpone development of the condition, disease or disorder. This delay can be of varying lengths of time, depending on the history of the condition, disease or disorder and/or individual being treated.

The condition, disorder or disease treated is an eosinophilic inflammatory or respiratory condition, disorder or disease.

The salmonid oil compositions provided herein improve anti-inflammatory efficacy via a reduction in eosinophil effector function. Thus, provided therein is a method for reducing eosinophil effector function in a human in need thereof, comprising administering the salmonid oil compositions provided herein, or compositions comprising biological active(s) isolated from the salmonid oil compositions provided herein, to the human to reduce eosinophil effector function.

### Sub-Patient Population

The methods provided herein involve treating a human in need thereof. In certain embodiments, the human is largely resistant to medical and surgical interventions for treating eosinophilic inflammatory conditions, disorders or diseases described herein. In one embodiment, the human exhibits or has resistance to steroid therapy. For example, in one variation, the human has steroid treatment resistant asthma.

In some variations of the foregoing, the human is a child. In certain variations, the human is less than 18 years old, less than 12 years old, less than 10 years old, less than 5 years old, less than 2 years old, or less than 1 year; or between 2 years old and 12 years old.

### Formulations

In some embodiments, the salmonid oil compositions provided herein, or compositions comprising biological active(s) isolated from the salmonid oil compositions provided herein, are formulated for oral administration. Forms suitable for oral administration may include, for example, tablets, pills, capsules, cachets, dragees, lozenges, liquids, gels, syrups, slurries, elixirs, suspensions, aerosols, or powders.

In certain embodiments, the pharmaceutical compositions described herein are in the form of syrups, capsules, and soft gels (including, for example, chewable gummies).

Techniques for formulation and administration of the compositions can be found in Remington's Pharmaceutical Sciences, 18th Ed.. Mack Publishing Co, Easton, Pa., 1990. The pharmaceutical compositions described herein can be manufactured using any conventional method, *e.g*., mixing, dissolving, granulating, dragee-making, levigating, emulsifying, encapsulating, entrapping, melt-spinning, spray-drying, or lyophilizing processes. An optimal pharmaceutical formulation can be determined by one of skill in the art depending on the route of administration and the desired dosage. Such formulations can influence the physical state, stability, rate of in vivo release, and rate of in vivo clearance of the administered agent.

In some variations, the salmonid oil compositions provided herein, or compositions comprising biological active(s) isolated from the salmonid oil compositions provided herein, are administered to the human as a unit dosage, for example, in the form of syrups, capsules, and soft gels (including, for example, chewable gummies) as described herein. In one variation, "unit dosage form" refers to physically discrete units, suitable as unit dosages, each unit containing a predetermined quantity of the salmonid oil compositions provided herein, or compositions comprising biological active(s) isolated from the salmonid oil compositions provided herein, which may be in a pharmaceutically acceptable carrier.

As used herein, by "pharmaceutically acceptable" is meant a material that is not biologically or otherwise undesirable. *e.g*., the material may be incorporated into a pharmaceutical composition administered to an individual without causing significant undesirable biological effects or interacting in a deleterious manner with any of the other components of the composition in which it is contained. Pharmaceutically acceptable carriers or excipients have preferably thus in some embodiments met the required standards of toxicological and manufacturing testing and/or are included on the Inactive Ingredient Guide prepared by the U.S. Food and Drug administration.

In some embodiments, the salmonid oil compositions provided herein, or compositions comprising biological active(s) isolated from the salmonid oil compositions provided herein, are formulated for inhalation. Forms suitable for inhalation may include, for example, dry powder, non-aqueous liquid formulation, or liquid formulation (*e.g*., for nebulization).

In some embodiments, the salmonid oil compositions provided herein, or compositions comprising biological active(s) isolated from the salmonid oil compositions provided herein, are formulated for injection. In some embodiments, formulations suitable for injection may comprise one or more aqueous or non-aqueous vehicles, one or more antimicrobials, one or more antioxidants, one or more buffers, one or more bulking agents, one or more chelating agents, one or more solubilizing agents, one or more surfactants, and/or one or more tonicity agents.

In some embodiments, the salmonid oil compositions provided herein, or compositions comprising biological active(s) isolated from the salmonid oil compositions provided herein, are formulated for topical administration. Forms suitable for topical administration may include, for example, solution, lotion, cream, ointment, gel, paste, aerosol foam or spray, powder, solid, or transdermal patch. In some embodiments, formulations suitable for topical administration may comprise water, oil, alcohol, propylene glycol, one or more preservatives, one or more emulsifiers, one or more absorption promoters, and/or one or more fragrances.

In some embodiments, the salmonid oil compositions provided herein, or compositions comprising biological active(s) isolated from the salmonid oil compositions provided herein, are administered orally, inhaled, injected or administered topically.

### Dosages

In some embodiments, the methods provided comprise administering to the human in need thereof an effective amount of the salmonid oil composition, or composition comprising biological active(s) isolated from the salmonid oil compositions provided herein. In some variations, an "effective amount" intends such amount of a composition or biological active of the invention which should be effective in a given therapeutic form. In certain variations, an effective amount of the salmonid oil composition, or composition comprising biological active(s) isolated from the salmonid oil compositions provided herein, is an amount sufficient to reduce eosinophil effector function in the human, and thereby treating the human suffering from the conditions, diseases or disorders described herein, or alleviating the existing symptoms of such conditions, diseases or disorders.

In some variations, exemplary dosage levels of the salmonid oil compositions provided herein for a human may be between 4g and 10g per day, or between 4g and 6g per day.

In some variations, exemplary dosage levels of the isolated biological active(s) from the salmonid oil compositions provided herein for a human may be between 10mg and 1000mg.

The final dosage regimen is determined by the attending physician in view of good medical practice, considering various factors that modify the action of the salmonid oil composition, or composition comprising biological active(s) isolated from the salmonid oil compositions provided herein, the identity and severity of the disease state, the responsiveness of the subject, the age, condition, body weight, sex, and diet of the subject, and the severity of any infection. Additional factors that can be taken into account include time and frequency of administration, drug combinations, reaction sensitivities, and tolerance/response to therapy. Further refinement of the dosage appropriate for treatment involving any of the formulations mentioned herein is done routinely by the skilled practitioner without undue experimentation, especially in light of the dosage information and assays disclosed, as well as the pharmacokinetic data observed in human clinical trials.

An effective amount may be in one or more doses. *i.e.,* a single dose or multiple doses may be required to achieve the desired treatment endpoint. In certain embodiments, the salmonid oil composition, or composition comprising biological active(s) isolated from the salmonid oil compositions provided herein, are administered once, twice, or three times daily. In certain embodiments, the salmonid oil composition, or composition comprising biological active(s) isolated from the salmonid oil compositions provided herein, are administered once or twice daily. In certain embodiments, the salmonid oil composition, or composition comprising biological active(s) isolated from the salmonid oil compositions provided herein, administered once daily.

### Articles of Manufacture and Kits

The salmonid oil composition, or composition comprising biological active(s) isolated from the salmonid oil compositions provided herein, that may be formulated in one or more pharmaceutically acceptable carriers, excipients or other ingredients can be prepared, placed in an appropriate container, and labeled for treatment of an indicated condition, disease or disorder. Accordingly, in certain aspects, also provided is an article of manufacture, such as a container comprising a dosage form of salmonid oil composition, or composition comprising biological active(s) isolated from the salmonid oil compositions provided herein, and a label containing instructions for use of such compositions or active(s).

In some embodiments, the article of manufacture is a container comprising a dosage form of salmonid oil composition, or composition comprising biological active(s) isolated from the salmonid oil compositions provided herein, and one or more pharmaceutically acceptable carriers, excipients or other ingredients, as disclosed in the appended claims. In one embodiment of the articles of manufacture described herein, the dosage form is a syrup, capsule and soft gel (including, for example, chewable gummies).

In certain aspects, kits also are provided, as disclosed in the appended claims. For example, in some embodiments, a kit can comprise a dosage form of salmonid oil composition, or composition comprising biological active(s) isolated from the salmonid oil compositions provided herein, and a package insert containing instructions for use of the composition/active(s) in treatment of a condition, disease or disorder. The instructions for use in the kit may be for treating an eosinophilic respiratory inflammation or inflammation of the respiratory tract, including, for example, asthma, bronchial asthma or acute respiratory syndrome.

The labels and package inserts of the articles of manufacture and kits, respectively, contain instructions for treating any of the conditions, diseases or disorders described herein, as disclosed in the appended claims. In some embodiments, the label contain instructions for treatment of eosinophilic inflammatory conditions, disorders or diseases, including respiratory conditions, disorders or diseases. In some variations, the label contain instructions for treatment of a chronic inflammatory disorder of the airways. In one variation, the label contain instructions for treatment of asthma, such as bronchial asthma and/or steroid treatment resistant asthma. In other embodiments, the label contain instructions for treatment of a viral respiratory disease, such as severe acute respiratory syndrome (including, for example, severe acute respiratory syndrome caused by a coronavirus).

It should be understood that any of the salmonid oil compositions or composition comprising biological active(s) isolated from the salmonid oil compositions described herein, and any of the conditions, diseases or disorders described herein, may be used with the various embodiments of the articles of manufacture and kits described herein, and falling within the scope of the appended claims.

### EXAMPLES

The presently disclosed subject matter will be better understood by reference to the following Examples, which are provided as exemplary of the invention, and not by way of limitation.

### Example 1

### Anti-inflammatory effects of enzymatically extracted salmonid oil

This example explores the anti-inflammatory effects via a reduction in eosinophil effector function in enzymatically extracted salmonid oil. The enzymatically extracted salmonid oil used in this example is Brilliant Salmon Oil (commercially available from Hofseth BioCare ASA), also referred to herein as "SO". Three in-vitro assays were used that measured (i) eosinophil shape change in normal polymorphonuclear leukocytes (PMNL), (ii) integrin upregulation in normal PMNL, and (iii) an apoptosis assay in allergic human purified peripheral blood eosinophils, to explore this hypothesis.

### Shape change assay

PMNL samples were pretreated with 3 µg/mL ApoA-IV (positive control), 100 µg/mL SO, and formulation vehicle (negative control) for 30 minutes and stimulated with serial dilutions of CCL11 for 20 minutes at 37 °C. Shape change was monitored by flow cytometry as the increase of forward scatter (FSC) and was expressed as percent of the vehicle response. Note, eosinophils are distinguishable from neutrophils by their SSC properties and autofluorescence.

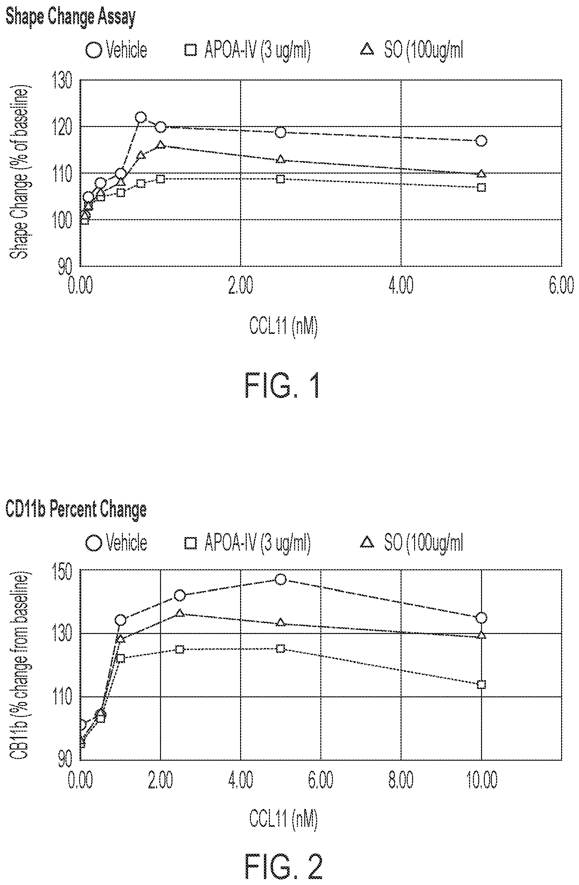

The chemotactic factor, CCL11, was used to prime eosinophils to immediately prepare for diapedesis through the endothelium by rearranging their cytoskeleton. This morphological change was detected by flow cytometry as an increase in the forward scatter properties of these cells. The effects of SO at 100 µg/mL was studied and compared to the known effect of 3 µg/mL ApoA-IV on eosinophil shape change in healthy donor PMNL samples. The pretreated samples were stimulated with serial dilutions of CCL11, and shape change was monitored by flow cytometry. As can be seen in Table 1 below and in FIG. **1**, 1 00 µg/mL of SO led to a visible decrease of eosinophil shape change as compared to vehicle.

**Table 1. Shape change (100% as baseline) of PMNL stimulated by varying concentrations of CCL11, with different pretreatment conditions.**

| **CCL11 (nM)** | **Vehicle (%)** | **3 µg/mL APOA-IV (%)** | **100 µg/mL SO (%)** |
|---|---|---|---|
| 0.05 | 101 | 100 | 101 |
| 0.10 | 105 | 103 | 103 |
| 0.25 | 108 | 105 | 106 |
| 0.50 | 110 | 106 | 108 |
| 0.75 | 122 | 108 | 114 |
| 1.00 | 120 | 109 | 116 |
| 2.50 | 119 | 109 | 113 |
| 5.00 | 117 | 107 | 110 |

### CD11b (integrin) upregulation assay

PMNL samples were pretreated with 3 µg/mL ApoA-IV (positive control), 100 µg/mL SO, and formulation vehicle (negative control) for 30 minutes and incubated with serial dilutions of CCL11 for 30 minutes at 37°C. Samples were stained with anti-CD16-PE-Cy5 and anti-CD11b-PE (ICRF44) antibodies. Eosinophils were identified as CD16 negative cells. CD11b upregulation was analyzed by flow cytometry.

A precondition for eosinophil migration is upregulation of adhesion molecules such as the αmβ2 integrins (CD11b/CD18). Similar to the effect observed on shape change above, SO and ApoA-IV clearly reduced the presence of CD11b molecules on the cell surfaces by 20-30% (at optimum around 2-4 nM CCL11 concentrations) as shown in Table 2 below and in FIG. **2****.**

**Table 2. CB11b change (100% as baseline) on PMNL surface stimulated by varying concentrations of CCL11, in different pretreatment conditions.**

| **CCL11 (nM)** | **Vehicle (%)** | **3 µg/mL APOA-IV (%)** | **100 µg/mL SO (%)** |
|---|---|---|---|
| 0.01 | 101 | 95 | 96 |
| 0.50 | 104 | 103 | 105 |
| 1.00 | 134 | 122 | 128 |
| 2.50 | 142 | 125 | 136 |
| 5.00 | 147 | 125 | 133 |
| 10.00 | 135 | 114 | 129 |

### Apoptosis assay

The allergic human eosinophils were placed in RPMI 1640 medium supplemented with IL-5 (50 pM). 1% FBS and PenStrep in the presence of 3 µg/mL ApoA-IV (positive control), 100 µg/mL SO, and formulation vehicle (negative control). Aliquots were removed after 18 hr incubation, washed twice in PBS, and resuspended in binding buffer. The eosinophil cells were stained using the Annexin V-FITC Apoptosis Detection Kit I, (Sigma Aldrich) and immediately analyzed by flow cytometry. Each sample was acquired for 1 min, and the total number of eosinophils gated on a forward scatter/side scatter plot and the percentage of live cells (annexin Vneg) and apoptotic cells (annexin Vpos) was recorded.

ApoA-IV and SO both accelerated eosinophil apoptosis in these allergic donor cells. The percentage of live cells (annexin Vneg) decreased from 57.6% ± 4.5 in the vehicle treatment to 31.5 ± 2.3% in 3 µg/mL ApoA-IV-treated eosinophils (positive control) and 41.6 ± 3.0% in 100 µg/mL SO-treated eosinophils. Both ApoA-IV and SO also increased the percentage of apoptotic cells (annexinVpos) from 44.1 ± 2.9% (vehicle treatment) to 60 ± 3.8% with 3 µg/mL ApoA-IV and 54.4 ± 2.5% with 100 µg/mL SO.

### Conclusion

The results of this example demonstrated that SO had potential therapeutic promise for the treatment of allergic and inflammatory conditions, particularly those involving eosinophil effector functions. Specifically, SO at 100 µg/mL was observed to inhibit eosinophil response to chemoattractant CCL11 in a Shape Change assay. Further, SO at 100 µg/mL was observed to inhibit eosinophil response to chemoattractant CCL11 in an integrin (CD11b) surface upregulation assay. Finally, SO was observed to enhance apoptosis in eosinophils sourced from allergic individuals.

### Example 2

### Effects of various processing conditions on the anti-inflammatory efficacy of enzymatically extracted salmonid oil

This example compared the effects of various processing conditions (*e.g.*, heating, acid and base treatment) on the anti-inflammatory efficacy of enzymatically extracted salmonid oil via a reduction in eosinophil effector function. The enzymatically extracted salmonid oil used in this example is Brilliant Salmon Oil (commercially available from Hofseth BioCare ASA), also referred to herein as "SO". The same three *in vitro* assays described in Example 1 above were used to explore the effect of processing treatments on SO, as well as to compare the effect against those of krill oil and standard 18/12 fish oil (referring to 18% DHA and 12% EPA content in the fish oil). The Krill oil and the standard 18/12 fish oil were Kirkland Signature Krill Oil and Kirkland Signature Wild Alaskan Fish Oil, both sourced from Costco, USA.

### Shape change assay

PMNL samples were pretreated with 100 µg/mL SO. heat, acid and base treated SO, krill oil, standard 18/12 fish oil, and formulation vehicle (negative control) for 30 minutes and stimulated with serial dilutions of CCL1 1 for 20 minutes at 37 °C. Shape change was monitored by flow cytometry as the increase of forward scatter (FSC) and was expressed as percent of the vehicle response. Eosinophils were distinguishable from neutrophils by their SSC properties and auto-fluorescence.

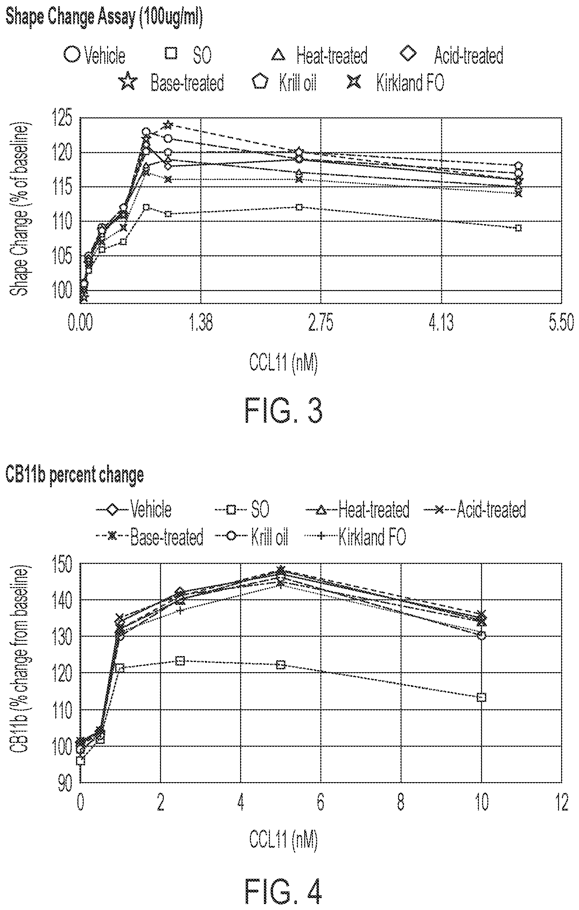

The chemotactic factor, CCL11, was used to prime eosinophils to immediately prepare for diapedesis through the endothelium by rearranging their cytoskeleton. This morphological change was detected by flow cytometry as an increase in the forward scatter properties of these cells. The effects of SO at 100 µg/mL was studied and compared to the various processing treatments on SO, krill oil and standard 18/12 fish oil on eosinophil shape change in healthy donor PMNL samples. The pretreated samples were stimulated with serial dilutions of CCL11, and shape change was monitored by flow cytometry. As can be seen in Table 3 below and in FIG. 3, the various processing treatments with heat, acid or base significantly reduced the efficacy of the SO in this assay. Further, krill oil showed no change from Vehicle. The sample of standard 18/12 fish oil showed some efficacy (-33% of the SO response) as compared to the formulation vehicle in this assay.

**Table 3. Shape change (100% as baseline) of PMNL stimulated by varying concentrations of CCL11. with different pretreatment conditions.**

| **CCL11 (nM)** | **Vehicle** | **SO** | **Heat-treated** | **Acid-treated** | **Base-treated** | **Krill oil** | **Standard 18/12 fish oil** |
|---|---|---|---|---|---|---|---|
| 0.05 | 101 | 101 | 100 | 100 | 99 | 101 | 100 |
| 0.10 | 105 | 103 | 104 | 104 | 105 | 104 | 104 |
| 0.25 | 109 | 106 | 108 | 109 | 108 | 109 | 107 |
| 0.50 | 111 | 107 | 112 | 111 | 112 | 112 | 109 |
| 0.75 | 123 | 112 | 118 | 121 | 122 | 120 | 117 |
| 1.00 | 122 | 111 | 119 | 118 | 124 | 120 | 116 |
| 2.50 | 119 | 112 | 117 | 119 | 120 | 120 | 116 |
| 5.00 | 117 | 109 | 115 | 116 | 116 | 118 | 114 |

### CD11b (integrin) upregulation assay

PMNL samples were pretreated with 100 µg/mL SO. heat, acid and base treated SO, krill oil, standard 18/12 fish oil, and formulation vehicle (negative control) for 30 minutes and incubated with serial dilutions of CCL11 for 30 minutes at 37°C. Samples were stained with anti-CD16-PE-Cy5 and anti-CD11b-PE (ICRF44) antibodies. Eosinophils were identified as CD16 negative cells. CD11b upregulation was analyzed by flow cytometry.

A precondition for eosinophil migration is upregulation of adhesion molecules such as the αmβ2 integrins (CD11b/CD18). Similar to the effect seen in Example 1 above, SO clearly reduced the presence of CD11b molecules on the cell surfaces by about 30% (at an optimum about 2-4 nM CCL11 concentration) whereas none of the other samples showed any difference from the formulation vehicle, as shown in Table 4 below and in FIG. **4****.**

**Table 4. CB11b change (100% as baseline) on PMNL surface stimulated by varying concentrations of CCL 11, in different pretreatment conditions.**

| **CCL11 (nM)** | **Vehicle** | **SO** | **Heat-treated** | **Acid-treated** | **Base-treated** | **Krill oil** | **Standard 18/12 fish oil** |
|---|---|---|---|---|---|---|---|
| 0.01 | 101 | 96 | 100 | 101 | 100 | 99 | 98 |
| 0.50 | 104 | 102 | 105 | 104 | 103 | 103 | 104 |
| 1.00 | 134 | 121 | 132 | 135 | 132 | 130 | 131 |
| 2.50 | 142 | 123 | 140 | 141 | 141 | 140 | 137 |
| 5.00 | 147 | 122 | 148 | 145 | 148 | 146 | 144 |
| 10.00 | 135 | 113 | 134 | 134 | 136 | 130 | 131 |

### Apoptosis assay

The allergic human eosinophils were placed in RPMI 1640 medium supplemented with IL-5 (50 pM), 1% FBS and PenStrep in the presence of 100 µg/mL SO, heat, acid and base treated SO, krill oil, standard 18/12 fish oil, and formulation vehicle (negative control). Aliquots were removed after 18 hr incubation, washed twice in PBS, and resuspended in binding buffer. The eosinophil cells were stained using the Annexin V-FITC Apoptosis Detection Kit I, (Sigma Aldrich) and immediately analyzed by flow cytometry. Each sample was acquired for 1 min, and the total number of eosinophils gated on a forward scatter/side scatter plot and the percentage of live cells (annexin Vneg) and apoptotic cells (annexin Vpos) was recorded.

SO again accelerated eosinophil apoptosis in these allergic donor cells as shown in Example 1 above. The percentage of live cells (annexin Vneg) decreased from 59.3% ± 4.1 in the vehicle treatment to 40.2% ± 3.8 in 100 µg/mL SO-treated eosinophils. SO also increased the percentage of apoptotic cells (annexinVpos) from 46.5% ± 2.7 (vehicle treatment) to 52.6% ± 3.8. None of the other treatments showed any significant change in percentage of live cells (annexin Vneg) from vehicle treatment. (Heat - 57.0% ± 3.8 ; Acid - 60.9% ± 3.3 ; Base - 59.5% ± 2.7 ; Krill - 53.2% ± 3.6 : Fish oil - 52.1% ± 4.3)

### Conclusion

The results of this example demonstrated that heating SO or contacting it with a mild acid or base reduces/removes its potential therapeutic application for the treatment of allergic and inflammatory conditions, particularly as shown in these eosinophil effector function *in vitro* results. These results show that heating or acid or base treatment significantly degrades/eliminates the active components present in SO which accounts for the positive eosinophil effector function results observed. Further, krill oil does not seem to contain these components, and the standard 18/12 fish oil may contain a very small amount of these components as shown by a slight positive result in the single shape change assay but an absence of response in the Cd11b upregulation or Apoptosis. Finally, without wishing to be bound by any theory, it the fatty acids present in the oils may not play a role in this efficacy since (a) no change in fatty acid composition was noted in SO after the heating or acid/base treatment and (b) fish oil and SO show a very similar fatty acid profile and are both in the natural triglyceride ester form.

### Example 3

### Comparative in-vivo study evaluating anti-inflammatory effects of enzymatically extracted salmonid oil

This example compares the effects of OmeGo^{®} Salmon oil (enzy matically extracted salmon oil commercially available from Hofseth BioCare ASA) with the effects of cod liver oil, Fevipiprant (as the positive control) and Linoleic acid (as the negative control) on chemotaxis and chemokinetics to leukotriene B4 and eosinophil viability in guinea pig peritoneal eosinophils.

The test items in this example include:
- OmeGo^{®} Salmon Oil (enzymatically extracted salmon oil commercially available from Hofseth BioCare ASA, also referred to herein as "OmeGo")
- SEACOD Cod Liver Oil (also referred to herein as "SEACOD")
- Fevipiprant; and
- Linoleic acid

The animals used in this example were from the species *Cavia porcellus* (Guinea Pig). A total of 21 male guinea pigs weighing between 200-250 g were selected. These animals were manually randomized into 7 groups i.e. G1 and G2 (OmeGo Salmon oil 30 and 300mg/kg). G3 and G4 (SEACOD 30 and 300 mg/ kg), G5 and G6 were positive control (Fevipiprant 5 and 20 mg/kg) and G7 was negative control (linoleic acid 300mg/kg) wherein each group consisted of 3 animals.

### Preparation of Test Item Formulation

OmeGo and SEACOD were encapsulated in capsules. The oil inside the capsule was collected using a syringe. Appropriate amount of the test item was weighed and suspended in the normal saline. At the time of dosing, the test item formulation was kept on magnetic stirrer to maintain homogeneity.

### General Method

Guinea pigs were sensitized to create mild eosinphilia by intraperitoneal injection of Polymyxin B (1 mg/animal) once a week for 6 weeks. Polymyxin B was given by dissolving it in 0.9 % saline solution. Each animal was injected with 0.5 mL of 0.9 % saline containing 1 mg of Polymyxin B. In week 6, in addition to the Polymyxin B, animals were injected with test items belonging to their specific group, as shown in Table 5 below.

**Table 5. Groups and Group Size**

| **Group No.** | **Treatment** | **Dose** | **No. of Animals** |
|---|---|---|---|
| G1 | OmeGo Salmon oil (CARDIO softgels) | 30 mg/kg | 03 |
| G2 | OmeGo Salmon oil (CARDIO softgels) | 300 mg/kg | 03 |
| G3 | SEACOD (Cod Liver Oil Capsule) | 30 mg/kg | 03 |
| G4 | SEACOD (Cod Liver Oil Capsule) | 300 mg/kg | 03 |
| G5 | Fevipiprant - PC | 5 mg/kg | 03 |
| G6 | Fevipiprant - PC | 20 mg/kg | 03 |
| G7 | Linoleic acid - NC | 300 mg/kg | 03 |

| | | | |
|---|---|---|---|
| **Key:** No. = Number, NC= Negative Control, PC - Positive Control. | | | |

### Method of Dosing and Collection of Intraperitoneal Fluid

Animals were treated with Polymyxin B through an intraperitoneal injection. Along with Polymyxin B even the test items were given through the intraperitoneal route. In the 6th week animals were anesthetized using isoflurane and the intraperitoneal fluid was collected by injecting 50 mL of saline in the peritoneum cavity. After massaging the abdomen for 15 seconds the fluid was drained and collected in the centrifuge tubes. Around 30-35 mL of fluid was collected from each animal. This fluid was centrifuged for 10 mins (400 x g 4°C) and was resuspended in FBS (Fetal bovine serum) medium. Following this step, the fluid was recentrifuged for 10 min (400 x g 4 °C) and the cell pellet was analyzed for chemotactic and chemokinetic responses of eosinophils to leukotriene B4 and cell counts using a 96-well microchemotaxis test chamber.

Eosinophils were recovered at greater than 90% purity using a modified procedure reported by Fukusa et. al. (Increased numbers of hypodense eosinophils in the blood of patients with bronchial asthma. Am. Rev. Respir. Dis. 1985, 132:981-985).

### Frequency of the Dosing

The animals were treated with Polymyxin B saline solution once every week for continuously 5 weeks and in the 6th week along with the Polymyxin B test item was given as per the groups. Each animal received 1 mg of Polymyxin B.

### Blood Collection

Blood was collected on previous day of first dosing and on the last day of the study. Through retro-orbital plexus around 1.5 mL of blood was collected for each animal and from this plasma was separated and stored at -20 °C for further anti-inflammatory biomarker assays.

### Observations

*Chemotactic and Chemokinetic Activities to Leukotriene B4:* The chemotactic and chemokinetic activities were measured by the Boyden chamber method using a 96-well microchamber and expressed as the number of eosinophils counted on the membrane filter under x400 magnification (cells/five fields). The chemokinetic activity to leukotriene B4 was examined by placing the same concentration of leukotriene B4 (0.1 µM) in both compartments of the chamber. Viability of the eosinophils was determined by the Trypan blue dye exclusion test after incubation for 90 min. All samples were assayed in duplicate. Blood was also collected on the same day and plasma was separated and stored at -20 °C for follow-on anti-inflammatory biomarker assays.

*Mortality*/*Morbidity*: All animals were observed at least twice daily (morning and evening) for morbidity and mortality, throughout the acclimatization and the study period.

*Clinical Signs of Toxicity*: Clinical signs were observed after test item administration and all the other days throughout the study period for all the animals. The cage side observations included (but not limited to) changes in skin and fur, eyes and mucous membranes, as well as respiratory, circulatory, autonomic and central nervous system, somatomotor activity and behavior pattern.

*Body Weight:* Animals were weighed during randomization, at start of treatment and weekly thereafter, until the end of experimental period.

*Evaluation of Results StatisticalAnalysis*: Raw Data were processed using Statistical Software Sigma Plot 14. The mean and Standard Deviation were calculated using the Software and all data were summarized in tabular form. All continuous data like the body weight was checked for their normality and for homogeneous data, ANOVA was used.

### Results and Discussions

*Chemotactic and Chemokinetic Activities to Leukotriene B4:* Data are presented for individual animals in each group in Table 6.

**Table 6. Summary of Pretreatment with Test Item on chemotactic (A) and chemokinetic (B) activities to leukotriene B4 (0.1 uM) in eosinophils obtained from guinea pig peritoneum.**

| **Group** | **Dose (mg/kg bw)** | **(A) Cells/5 fields** | | | **(B) Cells/5 fields** | | |
|---|---|---|---|---|---|---|---|
| **G1** | **30** | 465 | 460 | 470 | 240 | 235 | 225 |
| **G2** | **300** | 375 | 390 | 380 | 150 | 150 | 160 |
| **G3** | **30** | 745 | 730 | 720 | 340 | 325 | 350 |
| **G4** | **300** | 700 | 710 | 685 | 295 | 300 | 315 |
| **G5** | **5** | 425 | 390 | 420 | 290 | 280 | 290 |
| **G6** | **20** | 320 | 325 | 330 | 160 | 175 | 165 |
| **G7** | **300** | 770 | 790 | 760 | 335 | 360 | 340 |

*Mortality*/*Morbidity*: No treatment related mortality/morbidity were noted in any of the animals from all the 7 groups throughout the study period.

*Clinical Signs of Toxicity:* No treatment related clinical signs were observed in any of the groups.

*Body Weight and Body Weight Gain:* Mean body weights in the study showed no statistically significant difference in any of the groups throughout the study period (see Table 7). Percentage change in the body weight with respect to day 1 also showed no statistically significant difference in any of the groups throughout the study period (see Table 8).

**Table 7. Summary of Percent Change in Body weight with Respect to Day 1.**

| **Group** | **Dose (mg/kg of body weight)** | **Day** | **8** | **15** | **22** | **29** | **36** |
|---|---|---|---|---|---|---|---|
| **G1** | **30** | **Mean** | 10.38 | 18.82 | 31.90 | 46.53 | 62.71 |
| | | **SD** | 7.52 | 14.31 | 18.03 | 18.86 | 27.00 |
| **G2** | **300** | **Mean** | 4.68 | 8.37 | 27.10 | 35.37 | 44.82 |
| | | **SD** | 5.92 | 11.83 | 3.55 | 7.88 | 13.68 |
| **G3** | **30** | **Mean** | 9.70 | 13.56 | 29.23 | 32.91 | 45.32 |
| | | **SD** | 9.65 | 8.87 | 19.13 | 19.30 | 7.12 |
| **G4** | **300** | **Mean** | 6.68 | 12.30 | 19.36 | 32.88 | 44.94 |
| | | **SD** | 11.85 | 17.37 | 22.40 | 28.60 | 35.03 |
| **G5** | **5** | **Mean** | 14.31 | 14.49 | 36.25 | 49.30 | 57.70 |
| | | **SD** | 11.15 | 13.03 | 25.62 | 33.45 | 27.77 |
| **G6** | **20** | **Mean** | 7.95 | 15.74 | 31.78 | 45.78 | 59.50 |
| | | **SD** | 9.79 | 16.85 | 19.70 | 19.48 | 18.60 |
| **G7** | **300** | **Mean** | 17.89 | 19.38 | 25.44 | 39.07 | 48.73 |
| | | **SD** | 11.02 | 9.84 | 12.22 | 18.55 | 24.84 |

**Table 8. Summary of Mortality and Morbidity.**

| **Group** | **Dose (mg/kg bw)** | **No. of Incidence / No. of Animals** | **Observation** |
|---|---|---|---|
| **G1** | 30 | 03/03 | No mortality/ morbidity |
| **G2** | 300 | 03/03 | No mortality/ morbidity |
| **G3** | 30 | 03/03 | No mortality/ morbidity |
| **G4** | 300 | 03/03 | No mortality/ morbidity |
| **G5** | 5 | 03/03 | No mortality/ morbidity |
| **G6** | 20 | 03/03 | No mortality/ morbidity |
| **G7** | 300 | 03/03 | No mortality/ morbidity |

### Conclusion

Mild eosinophilia condition was created in all the animals using Polymyxin B. After administering the test item the Intraperitoneal fluid was successfully collected from all the animals in 6th week of the study.

The results of this example demonstrated that pretreatment with CARDIO oil (30 mg/kg i.p., n= 3; 300 mg/kg i.p., n = 3) significantly inhibited the eosinophil chemotactic and chemokinetic activities in a dose-dependent manner. Maximum inhibition (300 mg) was 50.7% for chemotaxis and 55.6% for chemokinesis, when compared to the linoleic acid negatice control. Pretreatment with SEACOD (30 mg/kg i.p., n = 3: 300 mg/kg i.p., n = 3) and linoleic acid (100 mg/kg i.p., n=3) did not inhibit eosinophil chemotactic and chemokinetic activities. Pretreatment with positive control Fevipiprant (5 mg/kg i.p., n=3: 20 mg/kg i.p., n=3) also significantly inhibited the eosinophil chemotactic and chemokinetic activities in a dose-dependent manner. Maximum inhibition (20mg) was 68.0% for chemotaxis and 51.7% for chemokinesis, when compared to control. The viability of eosinophils after a 90-min incubation with leukotriene B4 was greater than 96% in all treatment groups.

### Example 4

### Eosinophil modulating properties of OmeGo^{®} Salmon oil in House Dust Mite (HOM) extract-induced murine asthma model

This example demonstrates eosinophil-modulating properties of OmeGo^{®} Salmon oil (enzymatically extracted salmon oil commercially available from Hofseth BioCare ASA) in a House Dust Mite (HDM) extract-induced murine asthma model. For this purpose, normal saline (NS) was used as a vehicle control and Apolipoprotein A-IV (ApoA-IV) was used as a positive control, respectively. The study involved induction of asthmatic condition in mice using HDM extract and simultaneous evaluation of the effects of OmeGo Salmon oil (CARDIO softgels; 20 or 60 µg/animal) and ApoA-IV (5 µg/animal; as a positive control) on eosinophil modulation in mice.

No mortality or morbidity was observed during the course of the treatment. No clinical signs or symptoms were observed in any of the animals of all the groups. Animals across all the groups did not show any statistical variation in the body weight as well as in the % body weight change throughout the study period.

On day 1, all 20 mice were anesthetized and sensitized intranasally with 1 µg HDM protein in 40 µL PBS. From day 7 to day 11, all 20 mice were challenged by daily intranasal application of 10 µg HDM protein in 40 µL PBS. Mice were randomly divided into four groups with five mice in each group for differential treatment from day 7 to day 14. From day 7 to day 14, each animal in the first group (G1) received 100 µL NS; each animal in the second group (G2) received 20 µg CARDIO in 100 µL NS; each animal in the third group (G3) received 60 µg CARDIO in 100 µL NS; and each animal in the fourth group (G4) received 5 µg ApoA-IV in 100 µL NS.

The Broncho-Alveolar Lavage (BAL) fluid and spleen were collected from all 20 mice on day 15, and cellularity of the collected samples were analyzed by flow cytometry. As summarized in Table 9 below, total cell count (eosinophils, alveolar macrophages, lymphocytes, monocytes, and neutrophils) in BAL fluid significantly reduced in G3 (60 µg CARDIO/animal; P ≤ 0.01) and G4 (5 µg APOA-IV/animal: P ≤ 0.05) as compared to G1. However, significant reduction of the eosinophils in the BAL fluid was only observed in G3 (60 µg CARDIO/animal; P ≤ 0.01) as compared to G1. Further, percentage of eosinophils in spleen tissue significantly decreased in G3 (60 µg CARDIO/animal; P ≤ 0.001) and G4 (5 µg ApoA-IV/animal: P ≤ 0.01) as compared to G1. BAL fluid and spleen sample analysis showed that treatment of 60 µg CARDIO/animal helps in ameliorating airway eosinophilia in mice.

**Table 9. Summary of Eosinophilia Analysis**

| Group | Dose and material | | Cell count in BAL fluid | | Percent eosinophil in spleen tissue |
|---|---|---|---|---|---|
| | | | Total cell count | Eosinophil count | |
| G1 | Normal saline | Mean | 62740 | 37940 | 3.32 |
| | | SD | 12794 | 7284 | 0.32 |
| | | N | 5 | 5 | 5 |
| G2 | 20 µg/animal; CARDIO | Mean | 56660 | 40380 | 3.38 |
| | | SD | 7898 | 5391 | 0.50 |
| | | N | 5 | 5 | 5 |
| G3 | 60 µg/animal; CARDIO | Mean | 36480 | 21900 | 2.06 |
| | | SD | 8107 | 4285 | 0.29 |
| | | N | 5 | 5 | 5 |
| G4 | 5 µg/animal; APOA-IV | Mean | 41760 | 28220 | 2.34 |
| | | SD | 12536 | 8345 | 0.48 |
| | | N | 5 | 5 | 5 |

### Conclusion

Asthma condition was created in all the animals using HDM extract. After administering the test items, the BAL fluid was successfully collected from all the animals on the 15th day of the study. Administration of 60 µg CARDIO/animal was able to reduce the Eosinophilia condition in the murine asthma model, as reflected from the results of cellularity of the BAL fluid and spleen.

### Example 5

### Winterization of enzymatically extracted salmon oil and its effect on eosinophil apoptosis

This example compared the effects of winterization on the anti-inflammatory efficacy of enzymatically extracted salmonid oil via a reduction in eosinophil effector function. In a 100 mL glass beaker, 80 mL of OmeGo^{®} salmon oil (enzymatically extracted salmon oil commercially available from Hofseth BioCare ASA) (also referred to herein as "OmeGo") was placed, cooled to 8°C, and held between 4-8°C (refrigerator) for 24 hrs. A thin layer of waxy solid (about 10 mL in volume) precipitated at the bottom of the beaker. 10 mL of the top liquid oil was decanted and collected for eosinophil apoptosis assay as described below.

Allergic human eosinophils were placed in RPMI 1640 medium supplemented with IL-5 (50 pM), 1% FBS, and PenStrep together with the formulation vehicle, 100 µg/mL OmeGo, and 100 µg/mL winterized OmeGo from above. Aliquots were removed after an 18-hour incubation, washed twice in PBS, and resuspended in the binding buffer. The eosinophil cells were stained using the Annexin V-FITC Apoptosis Detection Kit I, (Sigma Aldrich) and immediately analyzed by flow cytometry. Each sample was acquired for 1 min, and the percentages of live cells (annexin Vneg) and apoptotic cells (annexin Vpos) were recorded, as summarized in Table 10 below.

**Table 10. Summary of apoptosis assay**

| Treatment | % Live cells | % Apoptotic cells |
|---|---|---|
| Formulation Vehicle | 58.1 | 40.6 |
| 100 µg/mL OmeGo | 40.0 | 56.3 |
| 100 µg/mL Winterized OmeGo | 55.4 | 42.9 |

### Conclusion

Winterization of OmeGO virtually eliminates eosinophil modulation as measured by increased apoptosis of eosinophil cells.

Unless clearly indicated otherwise. the terms "a," "an." and the like. refer to one or more.

In some variations, "about" refers to the usual error range for the respective value readily known to the skilled person in this technical field. Reference to "about" a value or parameter herein includes (and describes) embodiments that are directed to that value or parameter *per se.* For example, "about x" includes and describes "x" *per se.* In some embodiments, the term "about" when used in association with a measurement, or used to modify a value, a unit, a constant, or a range of values. refers to variations of +/- 2%.

In some variations, "between" two values or parameters herein includes (and describes) embodiments that include those two values or parameters per se. For example, description referring to "between x and y" includes description of "x" and "y'" per se.

## Claims

1. Composition comprising salmonid oil, wherein the salmonid oil is obtained from mild enzymatic hydrolysis of off-cuts of salmonid fish, for use in treating an eosinophilic inflammatory or eosinophilic respiratory condition, disorder, or disease in a human in need thereof, wherein the salmonid oil comprises gamma linolenic acid, columbinic acid, stearidonic acid, mead acid, and dihomo gamma linolenic acid.

2. Composition for use according to claim 1, wherein the eosinophilic inflammatory condition, disorder or disease is a chronic inflammatory disorder of the airways.

3. Composition for use according to claim 1, wherein the eosinophilic inflammatory condition, disorder or disease is asthma.

4. Composition for use according to claim 2, wherein the eosinophilic inflammatory condition, disorder or disease is bronchial asthma.

5. Composition for use according to claim 1, wherein the eosinophilic respiratory condition, disorder or disease is a viral respiratory disease.

6. Composition for use according to claim 1, wherein the eosinophilic respiratory condition, disorder or disease is severe acute respiratory syndrome.

7. Composition for use according to claim 6, wherein the severe acute respiratory syndrome is caused by a coronavirus.

8. Composition for use according to any one of the preceding claims, wherein the human is largely resistant to medical and surgical interventions for treating the condition, disorder or disease.

9. An article of manufacture, comprising:
a container comprising a composition according to anyone of claims 1 - 8, and
a label containing instructions for use directed to treatment of an eosinophilic inflammatory condition, disorder, or disease in a human in need thereof.

10. The article of manufacture of claim 9, wherein the composition is provided in a dosage form.

11. The article of manufacture of claim 10, wherein the dosage form is a syrup, chewable, capsule, or soft gel.

12. A kit, comprising:
a dosage form of a composition according to anyone of claims 1 - 8, and
a package insert containing instructions for use directed to treatment of an eosinophilic inflammatory condition, disorder, or disease in a human in need thereof.

13. The kit of claim 12, wherein the dosage form is a syrup, chewable, capsule or soft gel.

## Patentansprüche

1. Zusammensetzung, umfassend ein Salmonidenöl, wobei das Salmonidenöl aus mild enzymatischer Hydrolyse von Schnipseln von Salmoniden erlangt wird, zur Verwendung beim Behandeln einer eosinophilen entzündlichen oder eosinophilen respiratorischen Erkrankung, Störung oder Krankheit bei einem dessen bedürftigen Menschen, wobei das Salmonidenöl Gamma-Linolensäure, Columbinsäure, Stearidonsäure, Mead'sche Säure und Dihomogammalinolensäure umfasst.

2. Zusammensetzung zur Verwendung nach Anspruch 1, wobei die eosinophile entzündliche Erkrankung, Störung oder Krankheit eine chronische entzündliche Störung der Luftwege ist.

3. Zusammensetzung zur Verwendung nach Anspruch 1, wobei die eosinophile entzündliche Erkrankung, Störung oder Krankheit Asthma ist.

4. Zusammensetzung zur Verwendung nach Anspruch 2, wobei die eosinophile entzündliche Erkrankung, Störung oder Krankheit Bronchialasthma ist.

5. Zusammensetzung zur Verwendung nach Anspruch 1, wobei die eosinophile respiratorische Erkrankung, Störung oder Krankheit eine virale respiratorische Krankheit ist.

6. Zusammensetzung zur Verwendung nach Anspruch 1, wobei die eosinophile respiratorische Erkrankung, Störung oder Krankheit ein schweres akutes respiratorisches Syndrom ist.

7. Zusammensetzung zur Verwendung nach Anspruch 6, wobei das schwere akute respiratorische Syndrom durch ein Coronavirus verursacht ist.

8. Zusammensetzung zur Verwendung nach einem der vorhergehenden Ansprüche, wobei der Mensch gegen medizinische und chirurgische Eingriffe zum Behandeln der Erkrankung, Störung oder Krankheit großenteils resistent ist.

9. Herstellungsgegenstand, umfassend:
einen Behälter, umfassend eine Zusammensetzung nach einem der Ansprüche 1-8, und
ein Etikett, enthaltend Gebrauchsanweisungen, die auf eine Behandlung einer eosinophilen entzündlichen Erkrankung, Störung oder Krankheit bei einem dessen bedürftigen Menschen gerichtet sind.

10. Herstellungsgegenstand nach Anspruch 9, wobei die Zusammensetzung in einer Dosierungsform bereitgestellt ist.

11. Herstellungsgegenstand nach Anspruch 10, wobei die Dosierungsform ein Sirup, ein kaubares Produkt, eine Kapsel oder ein weiches Gel ist.

12. Kit, umfassend:
eine Dosierungsform einer Zusammensetzung nach einem der Ansprüche 1-8 und
eine Packungsbeilage, enthaltend Gebrauchsanweisungen, die auf eine Behandlung einer eosinophilen entzündlichen Erkrankung, Störung oder Krankheit bei einem dessen bedürftigen Menschen gerichtet sind.

13. Kit nach Anspruch 12, wobei die Dosierungsform ein Sirup, ein kaubares Produkt, eine Kapsel oder ein weiches Gel ist.

## Revendications

1. Composition comprenant de l'huile de salmonidé, dans laquelle l'huile de salmonidé est obtenue à partir d'une hydrolyse enzymatique douce de chutes de poissons salmonidés, destinée à être utilisée dans le traitement d'une affection, d'un trouble ou d'une maladie inflammatoire éosinophile ou respiratoire éosinophile chez un humain ayant besoin de celle-ci, dans laquelle l'huile de salmonidé comprend de l'acide gamma-linolénique, de l'acide columbinique, de l'acide stéaridonique, de l'acide hydrolytique et de l'acide dihomogamma-linolénique.

2. Composition destinée à être utilisée selon la revendication 1, dans laquelle l'affection, le trouble ou la maladie inflammatoire éosinophile est un trouble inflammatoire chronique des voies respiratoires.

3. Composition destinée à être utilisée selon la revendication 1, dans laquelle l'affection, le trouble ou la maladie inflammatoire éosinophile est l'asthme.

4. Composition destinée à être utilisée selon la revendication 2, dans laquelle l'affection, le trouble ou la maladie inflammatoire éosinophile est l'asthme bronchique.

5. Composition destinée à être utilisée selon la revendication 1, dans laquelle l'affection, le trouble ou la maladie respiratoire éosinophile est une maladie respiratoire virale.

6. Composition destinée à être utilisée selon la revendication 1, dans laquelle l'affection, le trouble ou la maladie respiratoire éosinophile est le syndrome respiratoire aigu sévère.

7. Composition destinée à être utilisée selon la revendication 6, dans laquelle le syndrome respiratoire aigu sévère est provoqué par un coronavirus.

8. Composition destinée à être utilisée selon l'une quelconque des revendications précédentes, dans laquelle l'être humain est largement résistant aux interventions médicales et chirurgicales pour traiter l'affection, le trouble ou la maladie.

9. Article manufacturé, comprenant :
un récipient comprenant une composition selon l'une quelconque des revendications 1 à 8, et une étiquette contenant des instructions d'utilisation destinées au traitement d'une affection, d'un trouble ou d'une maladie inflammatoire éosinophile chez un humain ayant besoin de celle-ci.

10. Article manufacturé selon la revendication 9, dans lequel la composition est fournie sous une forme posologique.

11. Article manufacturé selon la revendication 10, dans lequel la forme posologique est un sirop, un produit à croquer, une capsule ou un gel mou.

12. Kit comprenant :
une forme posologique d'une composition selon l'une quelconque des revendications 1 à 8, et
une notice d'emballage contenant des instructions d'utilisation destinées au traitement d'une affection, d'un trouble ou d'une maladie inflammatoire éosinophile chez un humain ayant besoin de celle-ci.

13. Kit selon la revendication 12, dans lequel la forme posologique est un sirop, un produit à croquer, une capsule ou un gel mou.
